# EUROPEAN PATENT APPLICATION

(11) **EP 3 017 839 A1**
(43) Date of publication of application: **11.05.2016**
(21) Application number: 15192026.1
(22) Date of filing: 29.10.2015
(51) Int. Cl.: A61M 11/06, A61M 11/00, A61M 15/00

(54) **A DEVICE FOR NEBULISING SUBSTANCES FOR AEROSOLS**

(30) Priority: 10.11.2014 IT VR20140279
(71) Applicant: Elettroplastica S.p.A., 25039 Travagliato (BS) (IT)
(72) Inventor: CHIARINI, Renato, 25039 TRAVAGLIATO (Brescia) (IT)
(74) Representative: Anselmi, Davide

(57) **Abstract**

Described is a device (1) for nebulising substances for aerosols comprising an outfeed duct (11) for the nebulised substance positioned in fluid communication between a nebulising chamber (C) and an outfeed section (11 b) of the outfeed duct (11) which can be used by a patient for inhaling operations, a separating element (12) configured to divide the nebulising chamber (C) in at least two separate sectors (C1, C2), and movement means (17) to move the separating element (12) in such a way as position at least partly at least one of the two sectors (C1, C2) facing at an infeed opening (11 a) of the outfeed duct (11) to adjust the flow rate of the fluid coming from the outfeed duct (11) of the device (1).

## Description

This invention relates to a device for nebulising substances for aerosols. More specifically, this invention can be used in the sector of apparatuses for nebulising a substance (usually of medical type) for aerosol therapy. The device is used by the patient for nebulising one or more liquid and/or solid substances, for example drugs, which must be inhaled in order to guarantee the therapeutic effectiveness.

More specifically, the device according to this invention is connected to an appliance which generates air under pressure in order to nebulise the substance to be inhaled in particles of predetermined dimensions of up to just a few microns. This substance usually comprises a drug in solution or in suspension. The nebulising device is able to nebulise the substance in such a way that it can be inhaled (through the nose and/or mouth) by a patient being treated.

A nebuliser for aerosols, better known as the device for nebulising or a nebuliser, comprises a lower portion forming a vessel for containing the substances to be nebulised and having a main opening. This lower portion also comprises a body projecting inside the containing vessel extending towards the main opening and comprising, in turn, inside it a slot for the passage of the air under pressure and connected, in use, to a unit for generating compressed air.

The projecting body has an outfeed hole for the pressurised air facing the main opening. Moreover, the device comprises a covering unit, also known as "pisper", surrounding the projecting body and forming a passage between it and the projecting body for the substance to be nebulised. Preferably, the pisper is positioned at a predetermined distance from the projecting body so as to form a hollow space for the passage of the substance to be nebulised. Alternatively, the passage may be defined by channels made in the inner surface of the pisper or on the outer surface of the projecting body. In addition, the pisper is slightly spaced from the bottom of the inhaler in such a way as to pass the substance to be nebulised towards the passage.

The pisper may be integral with the upper portion or be a separat piece which is mounted each time.

Thanks to the reduced dimensions of the outfeed hole relative to the inner slot, the outfeed speed of the pressurised air increases, pulling with it, by capillarity, small quantities of substance to be nebulised through the passage.

In other words, the pisper uses the Venturi Effect produced by a flow of air under pressure to nebulise the drug contained inside the inhaler.

In effect, there is generally a nebulising plane superposed over the outfeed hole of the projecting body and aimed at breaking up the particles of the liquid substance which reach it.

As mentioned, the device comprises an upper portion movable between a disconnected position wherein it is detached from the bottom portion and a connecting position wherein it is connected to the bottom portion at the main opening.

This upper portion comprises an air infeed duct positioned in fluid communication between the outside environment and the inhaler, during the connected position, for feeding atmospheric air in such a way as to satisfy the need for supplementary air. In addition, the device comprises an outfeed duct for the nebulised substance positioned in fluid communication between the inhaler, during the connected position, and an outfeed opening which can be connected to an accessory (such as, for example, a mask or a nasal adapter) for use by a patient.

In that way, the liquid substance nebulised passes through the outfeed duct in such a way to be reach the mouth or the nose of a user. This nebulised substance is also defined as aerosol and the relative nebulising device is also defined as aerosol device.

The dimensional characteristics of the particles which make up the aerosol considerably influence the effectiveness of the prescribed medial treatment; in effect, the diameter of the particles is inversely proportional to the capacity to penetrate into the bronchial tubes of the patient. The characteristics of a nebulising device are evaluated as a function of the size of the particles generated.

The particles are generally characterised by a MMAD (mass median aerodynamic diameter) of between 0.5 and 15 µm. From clinical studies and current regulations the particles generated by a nebuliser may be divided on the basis of the diameter and the corresponding type of depositing, as follows:
- between 0.5 and 3 µm there is alveolar deposition (low respiratory tracts),
- between 2 and 6 µm there is larynx-tracheal deposition (middle respiratory tracts),
- greater than 5 µm there is deposition in the upper respiratory tracts. Different solutions are known in the prior art for adjusting the size of the particles.

For example, in the majority of the known aerosols devices the air infeed duct may be replaced with a similar one of different dimensions to vary the capacity of the nebulisers of the drug to be inhaled. In effect, on the basis of the position of the air infeed duct relative to the outfeed hole of the projecting body it possible to guarantee a predetermined capacity for nebulising the substances to be nebulised.

The replacing of the infeed duct may, however, be slow and laborious, as well as the fact that the user must have several infeed ducts which can be fitted according to the specified nebulising requirements, based on the type of drug to be inhaled and/or the region of the bronchial tubes to be treated and reached.

Otherwise it is necessary to replace the infeed duct, as described in EP0653218. This document illustrates a nebulising device in which it is possible to use different air infeed ducts to meet the needs of different classes of dispersion of the drug and relative MMAD previously illustrated and allows a different nebulising of the substance introduced in the inhaler.

It is therefore necessary to have several end portions of the air infeed duct to obtain different nebulising effects.

Utility model M12009U000057 illustrates, on the other hand, a nebuliser equipped with a mechanism thanks to which the user can adjust the position of the air infeed duct relative to the outlet port of the nozzle, thus obtaining a greater or lesser nebulisation of the substances to be inhaled following the variation of the flow rate of air in the duct. The screening of the outlet port of the nozzle which emits the compressed air is performed by a suitable command located on the outside of the inhaler which vertically controls a cylinder. More specifically, the movement is performed by a rack integral with the outer surface of the infeed duct and an knob integral with the upper portion of the device equipped with toothing which meshes with the rack.

As well as the constructional difficulty of the knob-rack system, the main problem of this solution is that the aerosol may escape through the gaps created between the rack and the knob, thereby dispersing precious aerosol in the environment instead of channelling it in the outfeed duct from which the patient may inhale, also reducing the drawing effect of the outfeed duct. Other embodiments of nebulising devices are described in patent documents US5549102, GB 1283988, US 4674491.

In this context, the technical purpose which forms the basis of this invention is to propose a device for nebulising substances for aerosols which overcomes one or more of the above-mentioned drawbacks of the prior art.

More specifically, the aim of this invention to provide a device for nebulising substances for aerosols which is structurally simple, practical and versatile in use, allowing the user to adjust the nebulising capacity of the substance to be inhaled on the basis of the needs required by the medical therapy described.

A further aim of this invention is to provide a device for nebulising substances for aerosols which is efficient and guarantees a closing of the inner areas of the device in which the nebulised substance can expand, concentrating all the nebulised particles towards the outfeed duct and preventing losses.

The technical purpose indicated and the aims specified are substantially achieved by a device for nebulising substances for aerosols comprising the technical features described in one or more of the appended claims. More specifically, this invention comprises a device for nebulising substances for aerosols, comprising a first portion, a second portion, an infeed duct, an outfeed duct, a pisper and at least one separating element. The first portion has a lateral wall and a base defining a vessel for containing the substances to be nebulised and has a main opening opposite to the bottom of the containment vessel. The first portion also comprises a body projecting inside the containing vessel, extending towards the main opening and comprising inside it a slot for the transit of a pressurised fluid. The projecting body has an outfeed hole for the pressurised air facing the main opening.

The second portion defines a closing element for the main opening of the first portion and is movable between a disconnected position in which it is detached from the first portion and a connected position in which it is coupled to the first portion defining a chamber for nebulising the substance to be nebulised.

The air infeed duct for feeding atmospheric air extends between a first end section positioned in the nebulising chamber and a second end section positioned at the outside environment.

The outfeed duct for the nebulised substance positioned in fluid communication between the nebulising chamber and an outfeed section of the outfeed duct usable by a patient for inhalation operations.

Thea pisper surrounds the projecting body of the first portion and there is a passage between the pisper and the projecting body for passing the substance to be nebulised towards the pressurised air outfeed hole.

The passage is defined by a gap present between the pisper and the projecting body (in that case the pisper is positioned at a predetermined distance from the projecting body) or by channels made in the inner surface of the pisper or on the outer surface of the projecting body.

The separating element is positioned between the second end section of the infeed duct and the lateral wall of the containment vessel and is configured to divide the nebulising chamber in at least two separate sectors.

The device comprises movement means for moving the separating element in such a way as to position at least partly at least one of the two sectors facing the infeed opening of the outfeed duct to adjust the flow rate of the fluid flowing out from the outfeed duct of the device.

The dependent claims, incorporated herein for reference, relate to different embodiments of the invention.

Further features and advantages of the invention are more apparent in the non-limiting description which follows of a preferred embodiment of a device for nebulising substances for aerosols as illustrated in the accompanying drawings, in which:
- Figure 1 is a schematic exploded perspective view a device for nebulising substances for aerosols according to one possible embodiment of this invention,
- Figure 2 is a schematic perspective view partly in cross-section of the device of Figure 1 in a connection position,
- Figure 3A is a schematic cross-section of the device of Figure 1 along the line III-III in a connected position during a first operating configuration,
- Figure 3B is a schematic cross-section of the device of Figure 1 along the line III-III in a connected position during a second operating configuration, and
- Figure 4 is a schematic perspective view partly in cross section of a device of Figure 1 in the connecting configuration according to a possible alternative embodiment.

With reference to the accompanying drawings, the numeral 1 denotes in its entirety a device for nebulising substances for aerosols.

The device 1 comprises a first portion 2 comprising a lateral wall 2a and a bottom 2b, in use covered by the substances to be nebulised (not illustrated in the accompanying drawings), forming a containment vessel 3 for the substances to be nebulised, and comprising a main opening 4 opposite the bottom 2b of the containment vessel 3.

The first portion 2 comprises a body, not visible in the accompanying drawings, projecting inside the containing vessel 3, extending towards the main opening 4 and comprising inside it a slot 5 for the transit of a pressurised fluid.

Preferably, the slot passes through the bottom 2b of the containment vessel 3 and defines a duct 6 for connecting the first portion 2 with a unit for generating compressed air, not illustrated in the drawings.

Preferably the connecting duct 6 is positioned at the bottom 2b or at the lateral surface 2a of the first portion 2.

The projecting body has an outfeed hole 7 for the pressurised air facing the main opening 4.

In other words, the projecting body is raised above the bottom 2b towards the main opening 4 for a predetermined height. Preferably, the height of the projecting body is less than the plane positioned at the level of the main opening 4.

It should be noted that the fluid under pressure is preferably compressed air.

The outfeed hole 7 is positioned above the level of the substances and is positioned in fluid communication with the connecting duct 6 through the slot 5 of the projecting body.

Preferably, the outfeed hole 7 has a diameter less than the diameter of the slot 5 in such a way as to define an increase in the speed of the fluid flowing out (according to the known "Venturi Effect").

Thea pisper 8 surrounds the projecting body of the first portion 2 and there is a passage between the pisper 8 and the projecting body, not visible in the accompanying drawings, for passing the substance to be nebulised towards the pressurised air outfeed hole 7. As already mentioned, the passage is defined by a gap present between the pisper 8 and the projecting body (in that case the pisper 8 is positioned at a predetermined distance from the projecting body) or by channels made in the inner surface of the pisper 8 or on the outer surface of the projecting body. Preferably, the pisper 8 has an opening 8a at the outfeed hole 7 in such a way as to leave free the passage of air passing through it.

The substances to be nebulised are preferably in a liquid form and are positioned at a predetermined level inside the containment vessel 3. As already mentioned, the substances preferably contain a drug in solution or in suspension.

The device 1 comprises a second portion 9 defining an element for closing the main opening 4 of the first portion 2.

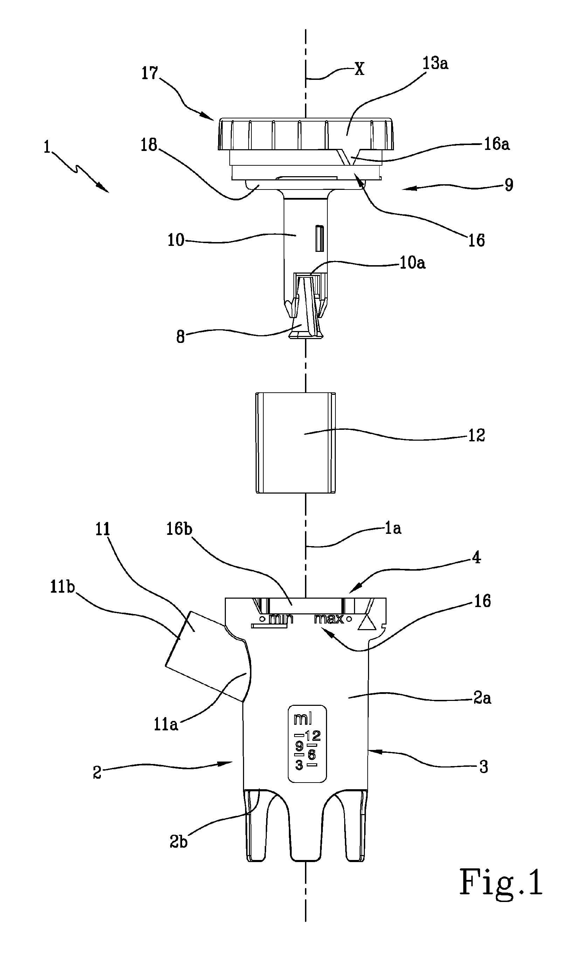

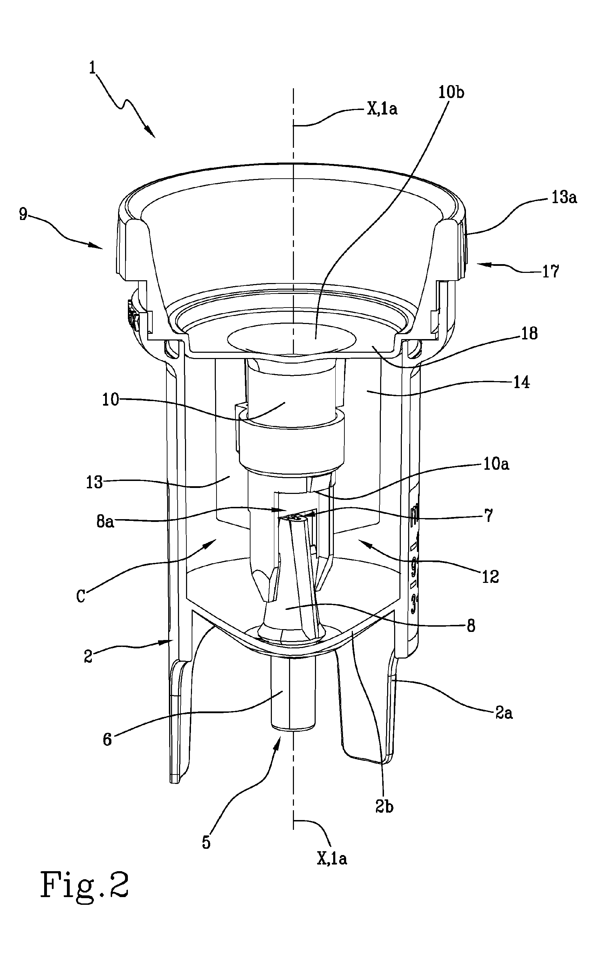

The a second portion 9 is movable between a disconnected position (Figure 1) in which it is detached from the first portion 2 and a connected position (Figure 2) in which it is coupled to the first portion 2 forming a chamber C for nebulising the substance to be nebulised.

More specifically, in the connected position, the second portion 9 is positioned above the first portion 2 with reference to the position wherein the device 1 is normally used as illustrated in Figure 2. The accompanying drawings show that the first portion 2 and the second portion 9 are positioned along a main axis 1 a of the device 1. Preferably, the main axis 1 a is vertical with reference to the position in which the device 1 is normally used.

The second portion 9 comprises a dividing wall 18 delimiting the top of the nebulising chamber C according the main axis 1 a positioned vertically. This dividing wall 18 divides the nebulising chamber C from the outside environment. Preferably this partition wall 18 is substantially horizontal. The device 1 comprises an air infeed duct 10 for feeding atmospheric air, extending between a first end section 10a positioned in the nebulising chamber C and a second end section 10b positioned at the outside environment.

Preferably the infeed duct 10 extends in a straight fashion along a main axis of extension X.

Preferably, the main axis of extension X of the infeed duct 10 is parallel to and/or coinciding with the main axis 1 a of the device 1.

Preferably, the second portion 9 comprises the infeed duct 10.

Preferably, the infeed duct 10 extends inside the chamber C. This infeed duct terminates inside the chamber C with a main opening facing the pisper 8 and located above it.

The separating wall surrounds the second end section 10b and extends from the latter towards the lateral wall 2a.

The device 1 according to this invention comprises an outfeed duct 11 for the nebulised substance, that is, the aerosol, positioned in fluid communication between the nebulising chamber C and an outfeed section 11 b of the outfeed duct 11 usable by a patient for inhalation operations. More specifically, the outfeed duct 11 has an infeed opening 11 a facing the nebulising chamber C of the first portion 2 so as to make a passage for the outlet of the nebulised substance (connectable to an accessory for use by a patient).

Preferably, by way of an example, the accessory for use by a patient may be a mask or a nasal adapter.

Preferably, the first portion 2 comprises the outfeed duct 11 and the infeed opening 11 a is positioned on the lateral wall 2a of the containment vessel 3.

Preferably, the outfeed duct 11 is positioned in a position alongside the infeed duct 10.

Alternatively, the second portion 9 comprises the outfeed duct 11 and the infeed opening 11 a is positioned at an upper wall of the second portion 9 alongside the end section 10b of the infeed duct 10.

The configuration of the parts described above allows the entrance of the compressed fluid (hereafter compressed air) in the containment vessel 3 through the outfeed hole 7. In addition, thanks to the reduced dimensions of the outfeed hole 7, the compressed air acquires a high speed in such a way as to draw by capillarity the substances contained in the containment vessel 3 through the passage between the pisper 8 and the containment vessel 3. In that way, the nebulising of the particles of the substance occurs at the outfeed hole 7. Preferably, the pisper 8 (or the device 1 itself) comprises in effect means for breaking up of the solution coming from the outfeed hole 7 of the projecting body and spaced from it by a predetermined distance in such a way that the nebulised solution makes contact with them.

Even more preferably, the breaking up means comprise a nebulising plane (of the type known in the sector) spaced from the outfeed hole 7 for breaking up the particles of nebulised liquid into even smaller fractions (the dimensions of these fractions of liquid particles being equal to a few micrometres). In the embodiment illustrated in the accompanying drawings, the nebulising plane is a single body with the pisper 8.

In this situation, the atmospheric pressure of the air entering through the infeed duct 10 pushes the particles towards the outfeed duct 11.

The pisper 8 is advantageously mounted centrally relative to the main axis 1 a in such a way as to enter into the projecting body, which is also positioned the centrally relative to the main axis 1 a.

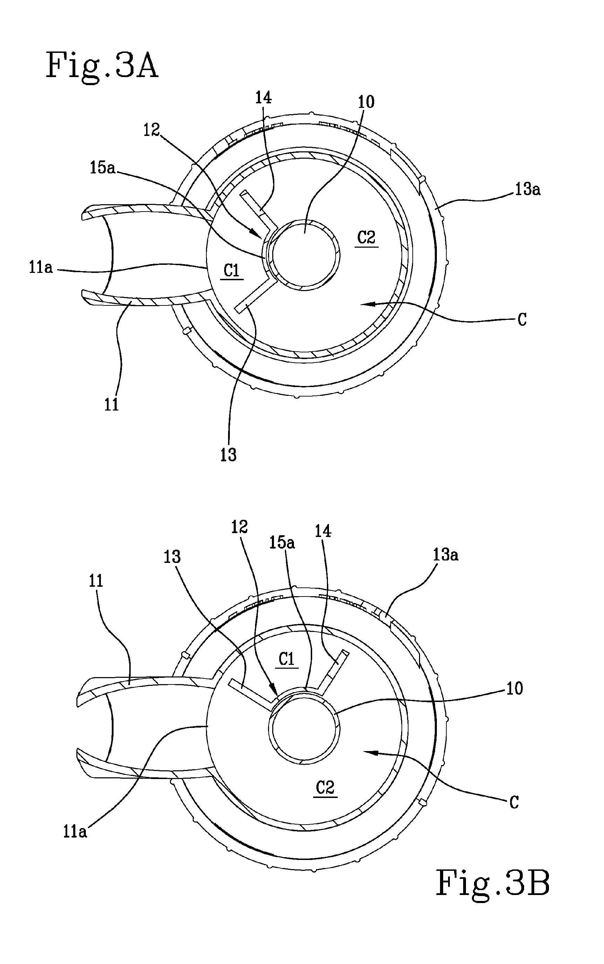

The device 1 comprises at least one separating element 12 positioned between the second end section 10b of the infeed duct 10 and the lateral wall 2a of the containment vessel 3 and is configured to divide the nebulising chamber C in at least two separate sectors C1, C2 (Figures 3A, 3B).

More specifically, in the embodiment illustrated in the accompanying drawings there are two sectors C1, C2.

Preferably, the two sectors C1, C2 delimit two different spaces inside the nebulising chamber C.

It should be noted that separating element 12 extends from the dividing wall 18 in the direction of the bottom 2b in such a way that the sectors C1, C2 are separate at this dividing wall 18. In other words, the element separating 12 is positioned alongside the dividing wall 18 (even if it is movable relative to the dividing wall).

Moreover, the separating element 12 is open in the direction of the bottom wall 2b in such a way that each sector C1, C2 is in fluid communication with the substance coming out from the outfeed hole 7. In other words, the separating element 12 does not comprise lower panels which divide at least one of the two sectors C1, C2 from the rest of the nebulising chamber C.

The device 1 comprises movement means 17 for moving the separating element 12 in such a way as to position at least partly at least one of the two sectors C1, C2 facing the infeed opening 11 a of the outfeed duct 11 to adjust the flow rate of the fluid flowing out from the outfeed duct 11 of the device 1.

Advantageously, the nebulising chamber C in which the aerosol expands is divided into two sectors C1, C2 by the separating element 12 which, moved by the movement means 17, allows adjustment inside the device 1 of the air flow rate through the infeed duct 10 and the flow rate of aerosol to be introduced in the outfeed duct 11 from which the user may inhale the nebulised substance.

In this way, the variation of the flow rate of fluid allows the dimensional properties of the particles to be altered, influencing as a result their penetrative properties into the respiratory tracts.

Preferably, the movement means 17 are defined by the second portion 9 mounted in a rotary fashion on the first portion 2 in the connected position. Still more preferably, the movement means 17 are configured to rotate the separating element 12 about the main axis X of extension of the infeed duct 10 and comprise a knob 13a rotatable together with to the infeed duct 10 about the axis of extension X.

Therefore, by simply rotating the separating element 12 the patient may advantageously perform a choking of the volume of the nebulising chamber C which is in direct contact with the infeed opening 11 a of the outfeed duct 11 in such a way as to induce a variation in flow rate of the aerosol which modifies the size of the particles of aerosol and the speed of the aerosol itself coming out from the outfeed device 1, in order to select which type of particles to inhale.

Preferably, the separating element 12 is operatively associated with the infeed duct 10 and even more preferably fixed to the infeed duct 10 itself. In the embodiment illustrated in the drawings, the separating element 12 is fixed to the infeed duct 10 by connect/disconnect means. Preferably, the connecting/disconnecting means comprise a ring which can be inserted around the infeed duct 10. However, in a possible embodiment of this invention the separating element 12 could be in one piece with the infeed duct 10 or in one piece with the first portion 2.

Preferably, the separating element 12 is positioned on the outside of the infeed duct 10. In other words, as shown clearly in Figures 3A and 3B, the separating element 12 is between the infeed duct 10 and the lateral wall 2a of the first portion 2 and extends towards the bottom 2b.

Preferably, the separating element 12 comprises at least a first panel 13 and a second panel 14 extending at least partly between the infeed duct 10 and the lateral wall 2a of the containment vessel 3 and at least partly between the second end section 10b of the infeed duct 10 and the bottom 2b of the containment vessel 3. More specifically, the two sectors C1, C2 are delimited by the first panel 13 and second panel 14.

In other words, the two panels 13, 14 divide the nebulising chamber C in two parts, that is to say, the sectors C1, C2.

As mentioned above, each panel 13, 14 extends from the dividing wall 18 (upper) in the direction of the bottom 2b. More specifically, each panel 13, 14 is placed alongside against the dividing wall 18.

Moreover, each of the sectors C1 and C2 are open at the bottom relative to the rest of the nebulising chamber C.

In a possible embodiment, the first panel 13 and second panel 14 are connected by an arc-shaped wall 15a in such a way as to form a single body preferably fixed on the infeed duct 10. Alternatively, the first panel 13 and second panel 14 may be connected directly to the infeed duct 10 or made in one piece with the infeed duct 10.

Preferably, least one of the two sectors C1, C2 extends between the first panel 13 and the second panel 14 in such a way as to completely cover the infeed opening 11 a of the outfeed duct 11 in at least one configuration for use of the device 1.

By positioning these sectors C1, C2 alternately or partly at the outfeed duct 11, it is possible to adjust the variation of the size of the particles of the aerosol fluid coming out from the containment vessel 3 and the quantity of nebulised substance.

The device 1 according to the invention in effect bases its operation on the variation of the volume of the nebulising chamber C using the panels 13, 14, and on the rotation of the separating element 12 inside it.

Preferably, the first panel 13 and second panel 14 lie on planes of extension passing through the main axis of extension X of the infeed duct 10. In other words, as in the embodiment illustrated in the accompanying drawings, the panels 13, 14 are vertical.

Preferably, the panels 13, 14 extend radially and rotate about the main axis 1 a of the device 1.

Figure 3A illustrates a configuration for use of the device 1 wherein the sector C1, having smaller dimensions than sector C2 (thus with smaller volume), is positioned at the opening 11 a of the outfeed duct 11 and it is the only one of the two sectors C1, C2 of the nebulising chamber C which communicates with the outfeed duct 11 and thus with the outside environment.

On the other hand, Figure 3B illustrates a configuration for use of the device 1 wherein the sector C2, having larger dimensions than sector C1 (thus with greater volume), is positioned at the opening 11 a of the outfeed duct 11 and it is the only one of the two sectors C1, C2 of the nebulising chamber C which communicates with the outfeed duct 11 and thus with the outside environment.

Advantageously, in the first configuration of use (Figure 3a) the dimensions of the particles nebulised are smaller, the quantity of particles is less and the speed of the fluid flowing out is higher, whereas in the second configuration of use (Figure 3b) the dimensions of the particles nebulised are larger, the quantity of particles is greater and the speed of the fluid flowing out is lower.

Advantageously, moreover, thanks to the movement means 17, and in particular in the preferred embodiment by using the knob 13a, it is possible to vary the mutual arrangement of the sectors C1, C2 and pass from the first to the second configuration gradually, obtaining a gradual variation of the effects of dimension, quantity and speed described above. There are in effect a plurality of intermediate configurations between the first and the second configuration, wherein both the sector C1 and the sector C2 partly face the infeed opening 11 a.

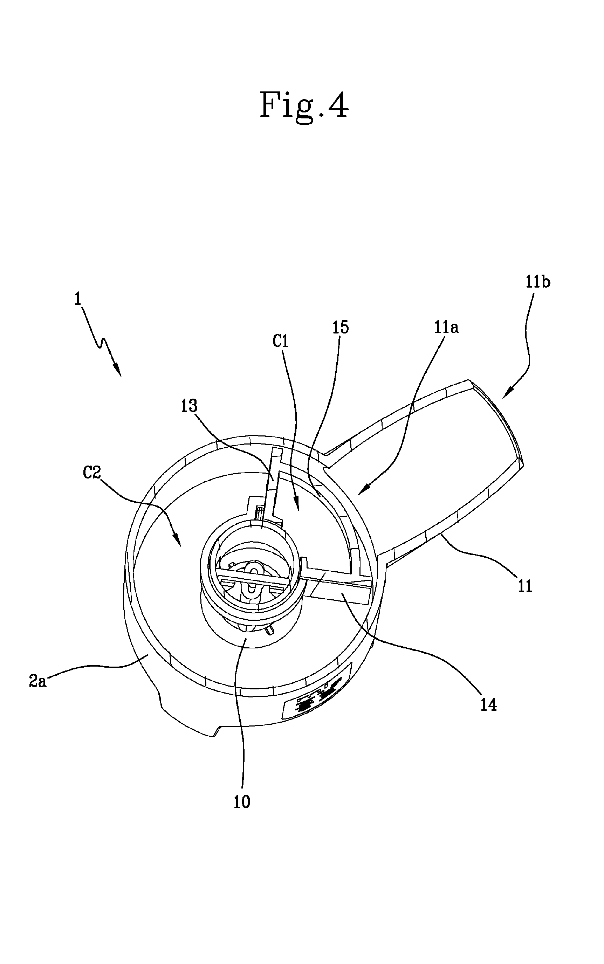

Preferably, according to a possible alternative embodiment of this invention illustrated in Figure 4, the separating element 12 comprises at least one wall 15, still more preferably curved, positioned close to the lateral wall 2a and extending between the two panels 13, 14 at least at one of the two sectors C1, C2. In the accompanying drawings the wall 15 is positioned at the smaller sector C1. Advantageously, the wall 15 allows a screen to be made designed to modify the flow rate of fluid coming from the outfeed duct 11 so as to obtain particular types of nebulising.

Moreover, according to a possible embodiment not illustrated in the accompanying drawings, the separating element 12 is preferably at least partly superposed on the pisper 8 at least at one of the two sectors C1 and C2. Preferably, the part of the separating element 12 superposed over the pisper 8 is the arc-shaped wall 15a. In this way, it is possible to make use completely of the power of the fine spray coming from the pisper 8 or some fractions of this fine spray.

The device 1 preferably comprises means 16 for indicating the mutual position of the first portion 2 and of the second portion 9 positioned at the outer surface of at least one of the two portions 2, 9, in such a way that they are visible to a user.

With reference to Figure 1, still more preferably the indicator means 16 comprise an arrow 16a placed on one of the two portions 2, 9 (in the preferred embodiment the arrow 16a is positioned on the knob 13a of the second portion 9) and a graduated scale 16b located on the other portion (in the preferred embodiment the graduated scale 16b is positioned near the edge of the first portion 2) indicating the point wherein by rotating the knob 13a the first configuration (Figure 3A) is reached and the point wherein the second configuration (Figures 3B) is reached, in such a way that the arrow 16a is slidable between the ends of the graduated scale 16b.

During assembly of the device 1, after having poured the substance to be nebulised in the containment vessel 3, the user firstly connects the pisper 8 (which, however, in a possible embodiment of this invention may be in one piece with one of the two portions 2, 9) superposing it on the projecting body 8 of the first portion 2 and connects the second portion 9 to the first portion 2 (in the case wherein the separating element 12 is already connected to the infeed duct 10 or as one with it).

After having connected a unit for generating compressed air to the connecting duct 6 and after having connected an accessory to the outfeed duct 11, it is possible to operate the generating unit to introduce compressed air in the slot 9 and thus trigger the step of nebulising the substance.

Before, after and during the nebulising, the user can adjust the degree of nebulising and the flow rate of aerosol produced towards the outfeed duct 11 by rotating the knob 13a, changing from the first configuration (Figure 3A) to the second configuration (Figure 3B). The use may also monitor the intensity of the variation of the two adjustments thanks to the indicator means 16.

The rotation of the knob 13a makes it possible to fully align, partly aligning or un-align the two sectors C1, C2 relative to the infeed opening 11 a.

This invention overcomes the drawbacks of the prior art thus allowing the dimensions of the particles to be increased or decreased in a simple and effective manner. In that way, it is possible to adjust the degree of nebulising as a function the type of treatment the patient must perform or as a function of the type of substance to be nebulised. Moreover, the possibility of manually controlling the adjustment of the degree of nebulising is performed by means of a simple rotation of the second portion 9 by the patient who may thereby personalise the relative therapeutic treatment in a practical and fast manner.

The particular construction technique and the juxtaposition of the components described so far guarantees, lastly, an effective seal and an almost hermetic closing of the chamber C which prevents any loss of precious aerosol obtained by the nebulising.

## Claims

1. A device for nebulising substances for aerosols, comprising:
- a first portion (2) comprising a lateral wall (2a) and a bottom (2b) forming a containment vessel (3) for the substances to be nebulised, and comprising a main opening (4) opposite and facing the bottom (2b) of the containment vessel (3); said first portion (2) comprising a body projecting inside the containing vessel (3), extending towards the main opening (4) and comprising inside it a slot (5) for the transit of a pressurised fluid; said projecting body comprising an outfeed hole (7) for the pressurised air opposite the main opening (4),
- a second portion (9) forming a closing element for the main opening (4) of the first portion (2) and being movable between a disconnected position in which it is detached from the first portion (2) and a connected position in which it is coupled to the first portion (2) forming a chamber (C) for nebulising the substance to be nebulised; said second portion (9) comprising a dividing wall (18) which delimits the nebulising chamber (C) in the direction of said second portion (9),
- an air infeed duct (10) for feeding atmospheric air, extending between a first end section (10a) positioned in the nebulising chamber (C) and a second end section (10b) positioned at the outside environment;
- an outfeed duct (11) for the nebulised substance positioned in fluid communication between said nebulising chamber (C) and an outfeed section (11 b) of the outfeed duct (11) usable by a patient for inhalation operations;
- a pisper (8) surrounding the projecting body of said first portion (2) and forming a passage between it and the projecting body for the substance to be nebulised towards the pressurised air outfeed hole (7);
- at least one separating element (12) positioned between the second end section (10b) of the infeed duct (10) and the lateral wall (2a) of the containment vessel (3) and configured for dividing the nebulising chamber (C) in at least two separate sectors (C1, C2);
**characterised in that** said separating element (12) is located at the dividing wall (18) and extends from the latter in the direction of the bottom (2b) in such a way that the sectors (C1, C2) are separate at said dividing wall (18); said separating element (12) being open in the direction of the bottom wall (2b) in such a way that each sector (C1, C2) is in fluid communication with the substance coming out from the outfeed hole (7);
the device 1 also comprises movement means (17) for moving the at least one separating element (12) in such a way as to position at least partly at least one of the two sectors (C1, C2) facing an infeed opening (11 a) of the outfeed duct (11) to adjust the flow rate of the fluid flowing out from the outfeed duct (11) of the device (1).

2. The device for nebulising substances for aerosols according to claim 1, wherein said infeed duct (10) extends in a straight fashion along its own main axis of extension (X).

3. The device for nebulising substances for aerosols according to claim 1 or 2, wherein said second portion (9) comprises said infeed duct (10).

4. The device for nebulising substances for aerosols according to one or more of the preceding claims, wherein said first portion (2) comprises said outfeed duct (11) and said infeed opening (11 a) is positioned on the lateral wall (2a) of the containment vessel (3).

5. The device for nebulising substances for aerosols according to one or more of claims 2 to 4, wherein said movement means (17) are designed to rotate said at least one separating element (12) around said main axis of extension (X) of said infeed duct (10).

6. The device for nebulising substances for aerosols according to one or more of the preceding claims, wherein said at least one separating element (12) is operatively associated with said infeed duct (10), preferably being fixed to the infeed duct (10).

7. The device for nebulising substances for aerosols according to one or more of the preceding claims, wherein said at least one separating element (12) comprises at least a first panel (13) and a second panel (14) extending at least partly between said infeed duct (10) and said lateral wall (2a) of the containment vessel (3) and at least partly between said second end section (10b) of the infeed duct (10) and said bottom (2b) of the containment vessel (3), said at least two sectors (C1, C2) being delimited by said first panel (13) and said second panel (14).

8. The device for nebulising substances for aerosols according to claim 7 when it depends on claim 2, wherein said first panel (13) and said second panel (14) lie in planes of extension passing through said main axis of extension (X) of the infeed duct (10).

9. The device for nebulising substances for aerosols according to one or more of claims 7 to 8, wherein at least one of the two sectors (C1, C2) extends between the first panel (13) and the second panel (14) in such a way as to completely cover the infeed opening (11 a) of the outfeed duct (11) in at least one configuration for use of the device (1).

10. The device for nebulising substances for aerosols according to one or more of the preceding claims, wherein said at least two sectors (C1, C2) delimit two different spaces inside the nebulising chamber (C).

11. The device for nebulising substances for aerosols according to one or more of claims 7 to 10 when dependent on claim 2, wherein said first portion (2) and said second portion (9) extend along a main axis (1 a) parallel to and/or coinciding with said main axis of extension (X) of the infeed duct (10) and wherein said panels (13, 14) extend radially and rotate around said main axis (1 a).

12. The device for nebulising substances for aerosols according to one or more of claims 7 to 11, wherein said separating element (12) comprises at least one wall (15), preferably curved, positioned close to the lateral wall (2a) and extending between the two panels (13, 14) at least at one of the two sectors (C1, C2).

13. The device for nebulising substances for aerosols according to one or more of claims 2 to 12, wherein said movement means (17) comprise a knob (13a) rotatable together with the infeed duct (10) around said main axis of extension (X).

14. The device for nebulising substances for aerosols according to one or more of the preceding claims, wherein said movement means (17) are formed by the second portion (9) rotatably mounted on said first portion (2) in the connected position.

15. The device for nebulising substances for aerosols according to one or more of the preceding claims, wherein said at least one separating element (12) is at least partly placed over said pisper (8).
